(19)

Europäisches
Patentamt
European
Patent Office
Office européen
des brevets

(11) **EP 3 977 936 A1**

(12) **EUROPEAN PATENT APPLICATION**
published in accordance with Art. 153(4) EPC

(43) Date of publication:
**06.04.2022 Bulletin 2022/14**

(21) Application number: **20823069.8**

(22) Date of filing: **28.05.2020**

(51) International Patent Classification (IPC):
**A61B 6/00** (2006.01)    **G01N 23/04** (2018.01)
**G01N 23/087** (2018.01)

(52) Cooperative Patent Classification (CPC):
**A61B 6/00; G01N 23/04; G01N 23/087**

(86) International application number:
**PCT/JP2020/021186**

(87) International publication number:
**WO 2020/250704 (17.12.2020 Gazette 2020/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **11.06.2019 JP 2019109018**

(71) Applicant: **Canon Kabushiki Kaisha
Tokyo 146-8501 (JP)**

(72) Inventors:
• **TERUI, Kosuke
Tokyo 146-8501 (JP)**

• **IWASHITA, Atsushi
Tokyo 146-8501 (JP)**
• **NODA, Takeshi
Tokyo 146-8501 (JP)**
• **TSUKUDA, Akira
Tokyo 146-8501 (JP)**
• **TORII, Sota
Tokyo 146-8501 (JP)**

(74) Representative: **WESER & Kollegen
Patentanwälte PartmbB
Radeckestraße 43
81245 München (DE)**

(54) **RADIOGRAPHIC IMAGING DEVICE AND RADIOGRAPHIC IMAGING METHOD**

(57)    An image processing apparatus that generates a material characteristic image with use of a plurality of radiation images that have been captured with different radiation energies, comprises a combining unit that combines radiation images based on two or more radiation images that are included among the plurality of radiation images, and combines radiation spectrums based on two or more radiation spectrums of the two or more radiation images, and a generating unit that generates a material characteristic image with use of two pairs of a radiation image and a radiation spectrum obtained from the plurality of radiation images. The generating unit uses, for at least one of the two pairs, a pair of the radiation image and the radiation spectrum combined by the combining unit.

**F I G. 5A**

```
           START
             │
      ┌──────────────┐
      │ OBTAIN IMAGES │──── S501
      └──────────────┘
             │
   ┌──────────────────┐
   │ OFFSET CORRECTION │──── S502
   └──────────────────┘
             │
   ┌──────────────────────┐
   │ DERIVE X-RAY SPECTRUMS │──── S503
   └──────────────────────┘
             │
   ┌──────────────────────────┐
   │ COMBINE SPECTRUMS/IMAGES  │──── S504
   └──────────────────────────┘
             │
   ┌──────────────────┐
   │ GAIN CORRECTION   │──── S505
   └──────────────────┘
             │
   ┌──────────────────────────┐
   │ COMPUTE SPECTRAL IMAGING  │──── S506
   └──────────────────────────┘
             │
           END
```

EP 3 977 936 A1

**Description**

TECHNICAL FIELD

The present invention relates to a radiation imaging apparatus and a radiation imaging method.

BACKGROUND ART

Today, radiation shooting apparatuses that use a flat panel detector (hereinafter abbreviated as FPD) formed with semiconductor materials have become widespread as shooting apparatuses used in radiation-based medical image diagnosis and nondestructive testing. In medical image diagnosis, for example, such radiation shooting apparatuses are used as digital shooting apparatuses for still image shooting such as general shooting, and for moving image shooting such as fluoroscopy shooting.

Applied technology of the FPD includes spectral imaging that makes use of energy information of radiation. In spectral imaging, a plurality of radiation images with different energies are obtained by, for example, emitting radiation multiple times under different tube voltages, and computation processing is performed with respect to these radiation images to obtain, for example, images from which arbitrary types of materials have been removed, area density images, and effective atomic number images. It is disclosed in PTL 1 that a weighted difference between the transmission intensity of low-energy X-rays and the transmission intensity of high-energy X-rays is obtained in order to determine the types of materials that compose a test subject with high precision.

CITATION LIST

PATENT LITERATURE

PTL 1: Japanese Patent Laid-Open No. 2017-122705

SUMMARY OF INVENTION

TECHNICAL PROBLEM

In spectral imaging, calculation is performed based on a plurality of radiation images with different energies and information of spectrums. In order to obtain images with favorable image quality through spectral imaging, it is necessary to obtain a more accurate calculation result; to this end, it is required that the energy difference between original images be large, and that information of radiation spectrums be accurate. However, due to restrictions on a shooting environment and a shooting method, such as a radiation generating apparatus, a subject, and a method of a detector, there are cases where it is difficult to obtain original images with a sufficient energy difference and high-precision spectrum information. Furthermore, although it is mentioned in PTL 1 that the precision of determination of the types of materials is improved by weighting the transmission intensities of X-rays, PTL 1 gives no consideration to radiation spectrums.

According to one aspect of the present invention, provided is a technique that makes it possible to provide a radiation image and a radiation spectrum that improve the precision or image quality of a generated material characteristic image.

SOLUTION TO PROBLEM

An image processing apparatus according to one aspect of the present invention has the following configuration. Specifically, an image processing apparatus generates a material characteristic image with use of a plurality of radiation images that have been captured with different radiation energies, and includes: combining means for combining radiation images based on two or more radiation images that are included among the plurality of radiation images, and combining radiation spectrums based on two or more radiation spectrums of the two or more radiation images; and generating means for generating a material characteristic image with use of two pairs of a radiation image and a radiation spectrum obtained from the plurality of radiation images, wherein at least one of the two pairs is a pair of the radiation image and the radiation spectrum combined by the combining means.

ADVANTAGEOUS EFFECTS OF INVENTION

According to the present invention, it is possible to provide a radiation image and a radiation spectrum that improve the precision or image quality of a generated material characteristic image.

Other features and advantages of the present invention will be apparent from the following description taken in conjunction with the accompanying drawings. Note that the same reference numerals denote the same or like components throughout the accompanying drawings.

BRIEF DESCRIPTION OF DRAWINGS

The accompanying drawings, which are incorporated in and constitute a part of the specification, illustrate embodiments of the invention and, together with the description, serve to explain principles of the invention.

FIG. 1 is a diagram showing an overall configuration of an X-ray imaging system according to a first embodiment.
FIG. 2 is a diagram showing examples of the spectrums of the energies of X-ray images.
FIG. 3 is a diagram showing an example of a relationship between X-ray energy and the number of photons.
FIG. 4A is a diagram for describing the average energy of an X-ray image.
FIG. 4B is a diagram for describing the average energy of an X-ray image.
FIG. 4C is a diagram for describing the average energy of an X-ray image.
FIG. 5A is a flowchart for describing processing performed by an imaging control apparatus.
FIG. 5B is a diagram for describing processing performed by an imaging control apparatus of the first embodiment.
FIG. 6 is a pixel circuit diagram of an X-ray imaging apparatus according to a second embodiment.
FIG. 7 is a timing chart of sampling and readout according to the second embodiment.
FIG. 8 is a diagram for describing processing performed by an imaging control apparatus of the second embodiment.

DESCRIPTION OF EMBODIMENTS

The following describes embodiments in detail with reference to the attached drawings. Note that the following embodiments do not limit the invention according to the claims. Although a plurality of features are described in the embodiments, not all of these plurality of features are indispensable for the invention, and furthermore, the plurality of features may be arbitrarily combined. Also, the same reference sign denotes the same or like constituents in the attached drawings, and redundant explanations are omitted.

Note, it is assumed that radiation according to the present invention includes not only $\alpha$-rays, $\beta$-rays, and $\gamma$-rays, and the like which are beams produced by particles (including photons) emitted through radiation decay, but also beams with energies of the same or higher level, such as X-rays, particle rays, cosmic rays, and the like. In the following embodiments, an apparatus that uses X-rays as one example of radiation will be described. Therefore, hereinafter, radiation, radiation images, radiation energy, a radiation spectrum, and a radiation dose will be described as X-rays, X-ray images, X-ray energy, an X-ray spectrum, and an X-ray dose, respectively.

<First Embodiment

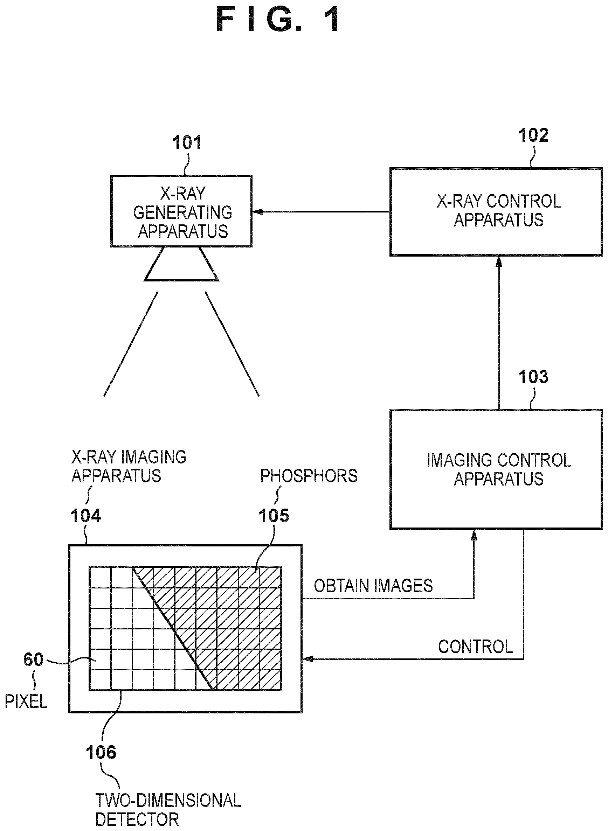

In a first embodiment, a configuration that increases the energy difference $\Delta E$ between X-ray images will be described. FIG. 1 is a block diagram showing a configuration of a radiation imaging system according to the first embodiment. The radiation imaging system includes an X-ray generating apparatus 101, an X-ray control apparatus 102, an imaging control apparatus 103, and an X-ray imaging apparatus 104, and performs imaging with use of X-rays.

The X-ray generating apparatus 101 generates X-rays, thereby exposing a subject thereto. The X-ray control apparatus 102 controls the generation of X-rays in the X-ray generating apparatus 101. The imaging control apparatus 103 includes, for example, one or more processors (CPUs) and a memory, and obtains X-ray images and performs image processing as the processor(s) executes a program stored in the memory. Note that each type of processing, including the image processing performed by the imaging control apparatus 103, may be realized by dedicated hardware, or may be realized by hardware and software operating in coordination with each other.

The X-ray imaging apparatus 104 includes phosphors 105 that convert X-rays into visible light, and a two-dimensional detector 106 that detects visible light. The two-dimensional detector 106 is a sensor in which pixels 60 that detect the X-ray quanta are disposed in an array of X columns and Y rows. The two-dimensional detector 106 converts the X-ray quanta detected by the pixels 60 into digital values with use of a non-illustrated AD converter, and transfers the digital values as an X-ray image to the imaging control apparatus 103. The imaging control apparatus 103 performs computation processing with respect to the X-ray image obtained from the two-dimensional detector 106, and generates a material separation image or a material identification image, which is a material characteristic image.

For example, the material separation image is obtained by setting a formula indicated by the following [Math. 1] on a per-energy basis, and solving the simultaneous equations.

[Math. 1]

$$I/I_0 = \frac{\int_0^\infty N(E)E\,exp\{-\mu_1(E)d_1 - \mu_2(E)d_2\}dE}{\int_0^\infty N(E)dE}$$

In [Math. 1], E denotes the energy of X-rays, N(E) denotes the spectrum of X-rays, $\mu_1(E)$ denotes a linear attenuation coefficient of a material 1, $\mu_2(E)$ denotes a linear attenuation coefficient of a material 2, $d_1$ denotes the thickness of the material 1, and $d_2$ denotes the thickness of the material 2. In [Math. 1], unknown variables are the thickness $d_1$ and the thickness $d_2$. By assigning captured images based on X-rays with two different energies to [Math. 1], two independent formulae can be generated; thus, the values of the thickness $d_1$ and the thickness $d_2$ can be obtained by solving these two independent formulae.

On the other hand, the material identification image (effective atomic number image, area density image) can be obtained by using the following [Math. 2].

[Math. 2]

$$I/I_0 = \frac{\int_0^\infty N(E)E\,exp\{-\mu(Z_{eff}, E)D_{eff}\}dE}{\int_0^\infty N(E)dE}$$

In [Math. 2], E denotes the energy of X-rays, N(E) denotes the spectrum of X-rays, $\mu(Z_{eff}, E)$ denotes a mass attenuation coefficient under the effective atomic number $Z_{eff}$ and energy E, and $D_{eff}$ denotes an effective area density. In [Math. 2], unknown variables are the effective atomic number $Z_{eff}$ and the effective area density $D_{eff}$. Therefore, similarly to the case where the aforementioned material separation image (the spatial distribution of thickness per material) is obtained, two independent formulae are generated by assigning captured images obtained based on X-rays with two different energies to [Math. 2]. The values of the effective atomic number $Z_{eff}$ and the effective area density $D_{eff}$ can be obtained by solving these two independent formulae.

Both [Math. 1] and [Math. 2] calculate spectrum space information of the spectrum N(E) of X-rays. At this time, if the difference between two different X-ray energies is large and spectrum information is accurate, high-precision calculation can be performed, and a favorable material separation image and material identification image can be generated.

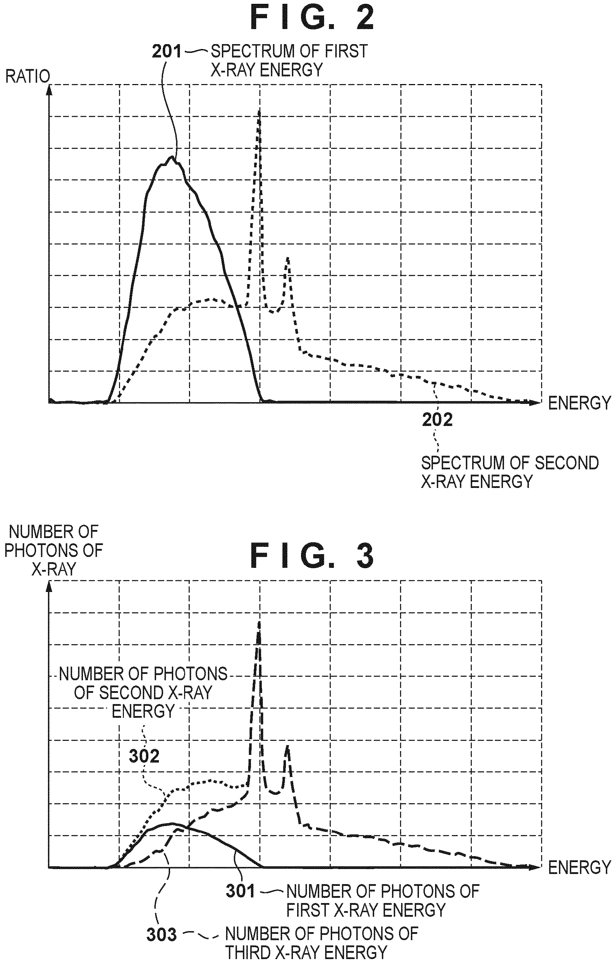

FIG. 2 shows the spectrums of X-ray energies according to the first embodiment. It is assumed that an X-ray image captured with a first X-ray energy is $X_1$, and an X-ray image captured with a second X-ray energy is $X_2$, where the second X-ray energy > the first X-ray energy. The spectrum of the X-ray energy of the X-ray image $X_1$ and the spectrum of the X-ray energy of the X-ray image $X_2$ are a first X-ray spectrum 201 and a second X-ray spectrum 202, respectively.

In the first embodiment, an X-ray image $X_3$ corresponding to a third X-ray energy with a third X-ray spectrum is generated by combining the X-ray image $X_1$ and the X-ray image $X_2$ together, and the first X-ray spectrum 201 and the second X-ray spectrum 202 together. The present embodiment will be described in relation to a case where the X-ray image $X_1$ and the X-ray image $X_2$ are combined based on $X_3 = X_2 - \alpha X_1$ with use of a predetermined coefficient $\alpha$. Note that $\alpha$ is an arbitrary coefficient.

In combining the X-ray images and the X-ray spectrums, it is necessary that the pixel values of the X-ray images have a correlation with the X-ray spectrums. While the pixel values of the X-ray images are information indicating the energies and doses of X-rays, the spectrums are information indicating the rates (distribution) of X-ray energies. Therefore, the X-ray spectrum of the post-combination X-ray image ($X_3$), which has been obtained through addition/subtraction performed with respect to the X-ray images (X-ray images $X_1$ and $X_2$), cannot obtained simply by performing addition/subtraction with respect to the spectrums (X-ray spectrums 201 and 202). For this reason, in the image processing of the present embodiment, in order to obtain the third X-ray spectrum corresponding to the post-combination X-ray image $X_3$, the X-ray spectrum 201 and the X-ray spectrum 202 are converted into the numbers of X-ray photons.

FIG. 3 shows an example of a relationship between X-ray energy and the number of photons according to the first embodiment. The numbers of photons of the X-ray spectrums of the X-ray image $X_1$ based on the first X-ray energy and the X-ray image $X_2$ based on the second X-ray energy are indicated by 301 and 302, respectively. The numbers of photons 301 and 302 have a correlation with the doses for the X-ray images $X_1$ and $X_2$ at the time of imaging. If the doses increase or decrease, the numbers of photons, too, consequently increase or decrease while maintaining the energy spectrums. The number of photons 303 is the number of photons obtained by combining the number of photons 301 and the number of photons 302. The number of photons 303 is obtained by performing addition/subtraction with respect to the number of photons 301 and the number of photons 302 similarly to the addition/subtraction that has been performed to obtain the X-ray image $X_3$ from the X-ray image $X_1$ and the X-ray image $X_2$. That is to say, in the first embodiment, the number of photons 303 is obtained by subtracting "the product of the number of photons 302 and $\alpha$" from "the number of photons 301". Then, a third spectrum is obtained by performing standardization so that the integrated value of the number of photons 303 coincides with the integrated value of the X-ray spectrum 201 (= the integrated value of the X-ray spectrum 202).

Note that the estimated values calculated from information of the doses and the spectrums of X-ray energies (201 and 202) at the time of capturing of X-ray images can be used as the numbers of photons 301 and 302 of the X-rays of the X-ray image $X_1$ based on the first X-ray energy and the X-ray image $X_2$ based on the second X-ray energy. A dose is the product of the energy × the number of photons of each X-ray, and the number of photons of each energy is proportional to the spectrum of X-rays; thus, the number of photons can be estimated from information of the dose and the spectrum. Alternatively, actual measured values obtained by performing measurement with use of, for example, a spectrometer may be used. Furthermore, the coefficient $\alpha$ is an arbitrary value, and is set so as to become close to, for example, the spectrum of desired energy. At this time, it is preferable to set the coefficient $\alpha$ in a range in which the post-combination spectrum does not take a negative value.

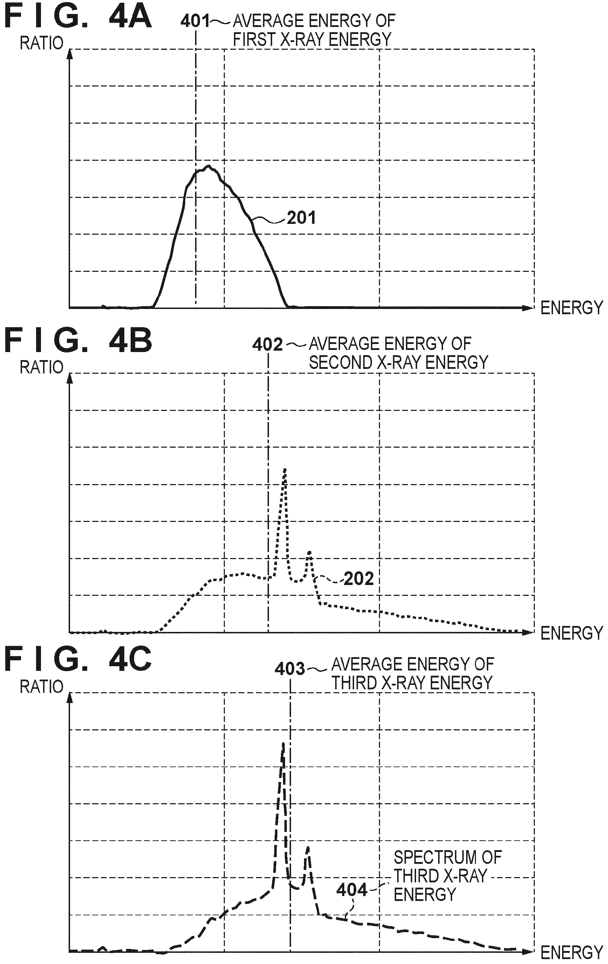

FIGs. 4A to 4C show the average energies of the X-rays of the images $X_1$ to $X_3$ according to the first embodiment. FIG. 4A shows the average energy 401 of the X-ray image $X_1$ captured with the first X-ray energy. FIG. 4B shows the average energy 402 of the X-ray image $X_2$ captured with the second X-ray energy. FIG. 4C shows the average energy 403 of the third X-ray energy corresponding to the combined X-ray image $X_3$. The magnitude relationship among these average energies is [the average energy 403] > [the average energy 402] > [the average energy 401]; the average energy 403 corresponding to the X-ray spectrum 404 of the third X-ray energy generated through the combining is the highest.

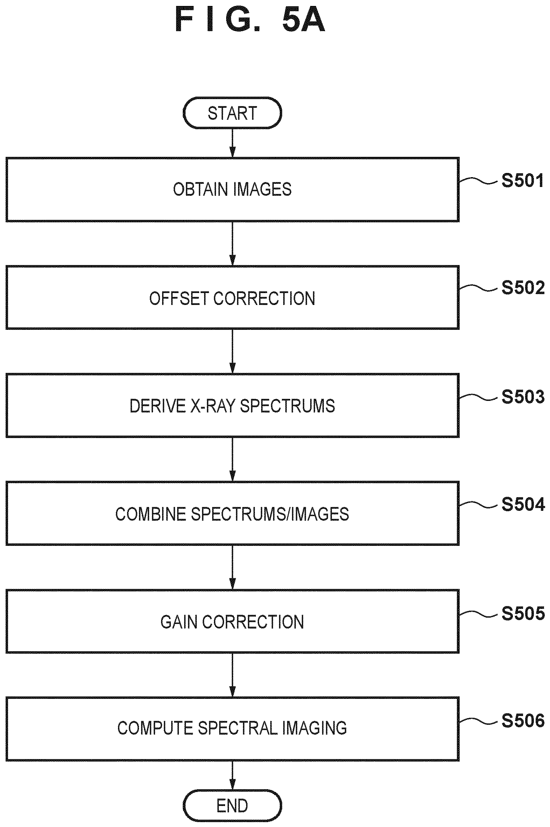

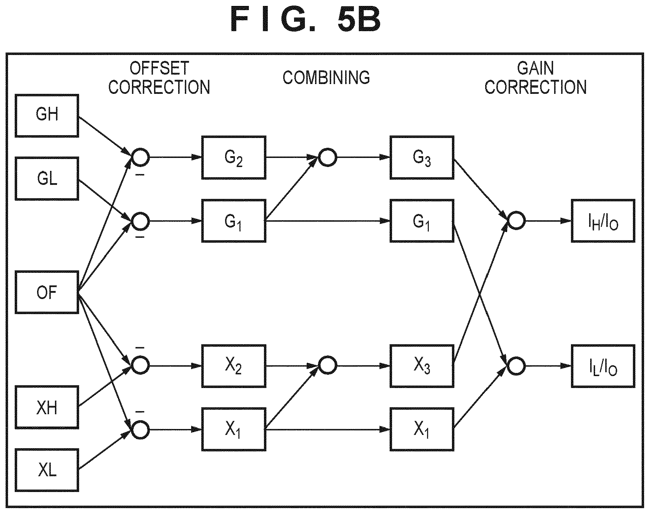

FIG. 5A is a flowchart showing processing performed by the imaging control apparatus 103 of the first embodiment to generate a material characteristic image. Also, FIG. 5B is a diagram for describing obtained images and processing applied to the images. In step S501, the imaging control apparatus 103 obtains X-ray images (XH and XL) with two different types of X-ray energies from the X-ray imaging apparatus 104 by controlling the X-ray generating apparatus 101 via the X-ray control apparatus 102. Here, the imaging control apparatus 103 also obtains an offset image (OF) captured in a state where there is no exposure to X-rays, as well as two gain images (GH and GL) captured through emission of X-rays with the two types of X-ray energies in a state where there is no subject. In step S502, the imaging control apparatus 103 obtains the differences between the two X-ray images captured with the two types of X-ray energies and the offset image, thereby obtaining the X-ray image $X_1$ (= XL - 0F) and the X-ray image $X_2$ (= XH - 0F) from which offset components have been removed. Note that although the foregoing has described an example in which the same offset image (OF) is used, no limitation is intended by this. It is permissible to obtain and use offset images (OF$_{GH}$, OF$_{GL}$, OF$_{XH}$, and OF$_{XL}$) that respectively correspond to the gain images and the X-ray images (GH, GL, XH, and XL).

In step S503, the imaging control apparatus 103 obtains the X-ray spectrums of the two types of X-ray energies. At this time, information of the X-ray energies that were emitted in the obtainment of the X-ray images is necessary. Such information of the X-ray energies can be obtained from, for example, tube voltages that were set by the X-ray control apparatus 102 with respect to the X-ray generating apparatus 101. Alternatively, the information may be estimated from the material transmission amounts in the X-ray images. Furthermore, although literature values may be used as the X-ray spectrums, it is preferable to use actual measured values obtained by measuring the X-rays generated by the X-ray generating apparatus 101 with use of, for example, a spectrometer.

In step S504, the imaging control apparatus 103 combines the X-ray image $X_1$ and the X-ray image $X_2$ and combines the X-ray spectrums with use of the method described using FIG. 2 to FIG. 4C, thereby obtaining the combined X-ray image $X_3$ and third X-ray spectrum 404. In step S505, the imaging control apparatus 103 performs gain correction in which gain variations are corrected on a per-pixel basis by dividing the X-ray images $X_1$ and $X_3$ by the gain images (Gi, $G_3$) obtained with their respective X-ray energies. Note that the gain image $G_1$ used in this processing is a gain image that has undergone the offset correction (GL - OF) in processing of step S502. Also, the gain image $G_3$ corresponding to the third X-ray spectrum 404 has undergone the offset correction (GH - OF) in processing of steps S502 to S504, and is generated by the aforementioned combining processing ($G_3 = G_2 - \alpha G_1$) with use of the gain images (Gi and $G_2$) corresponding to the two types of X-ray energies.

In step S506, the imaging control apparatus 103 performs computation of spectral imaging with use of two images after the gain correction (attenuation rate images $I_H/I_0$ and $IL/Io$). That is to say, the imaging control apparatus 103 obtains a material separation image or a material identification image by solving the simultaneous equations that are obtained by assigning, to [Math. 1] or [Math. 2], the pixel values and the spectrums of X-ray images with a plurality of different energies.

Here, when setting the formulae for the two different X-ray energies with use of [Math. 1] or [Math. 2], the imaging control apparatus 103 uses the X-ray image $X_3$ and the third X-ray spectrum corresponding to the third X-ray energy, which have been derived through the combining, as a high-energy X-ray image. Furthermore, the X-ray image obtained by applying the offset correction and gain correction to the X-ray image obtained with use of the first X-ray energy is used as a low-energy X-ray image. In this way, the X-ray energy difference between the high-energy X-ray image and the low-energy X-ray image used in the generation of the material characteristic image can be increased. As stated earlier, when the energy difference between the X-ray images used in the generation of the material characteristic image is large, the precision of calculation increases, and it is expected that the precision of material separation be improved, and the image quality be improved due to a reduction in noise of an effective atomic number image and an area density image.

The foregoing has described an example in which the third X-ray image and X-ray spectrum are combined based on X-ray images with two different X-ray energies and the X-ray spectrums thereof, and two simultaneous equations are set using the post-combination X-ray image and X-ray spectrum and one of the two original X-ray images. That is to say, the present embodiment has been described using an example in which the combined image $X_3$ and the X-ray image $X_1$ with the first X-ray energy are used in the generation of the material characteristic image. However, the X-ray images used in the computation of spectral imaging (the generation of the material characteristic image) is not limited to these. For example, an image that has been shot with another X-ray energy that is different from the first and second X-ray energies may be used as the low-energy X-ray image in generating the material characteristic image. This case corresponds to a mode in which two simultaneous equations are set from images with three different X-ray energies. As a result of solving the simultaneous equations in this mode, computation is performed using a large number of X-ray images compared to a case where two simultaneous equations are set from two different X-ray energies, and it is therefore expected that a result with higher precision be achieved. Furthermore, if an X-ray image with X-ray energy lower than the first X-ray energy and the combined image are used in the generation of the material characteristic image, the difference between X-ray energies can be further increased.

Furthermore, although the X-ray images and the X-ray spectrums of two different X-ray energies are combined in the first embodiment, the X-ray images and the X-ray spectrums of three or more different X-ray energies may be combined. In addition, simultaneous equations may be set with respect to the X-ray images with three or more different X-ray energies. By adopting this mode, for example, the number of materials to be separated can be increased.

As described above, the imaging control apparatus 103 of the first embodiment combines radiation images based on two or more radiation images included among a plurality of radiation images, and combines radiation spectrums based on the radiation spectrums of these two or more radiation images. Then, the imaging control apparatus 103 performs computation processing of spectral imaging, in which a material characteristic image is generated by solving the equations indicated by, for example, [Math. 1] and [Math. 2] with use of two pairs of a radiation image and a radiation spectrum obtained from the plurality of radiation images. Here, at least one of the two pairs of the radiation image and the radiation spectrum used in the computation is a pair of the combined radiation image and radiation spectrum described above. The pair of the combined radiation image and radiation spectrum is, for example, an X-ray spectrum with higher average energy and a corresponding X-ray image; thus, a larger energy difference can be ensured between the X-ray images used in spectral imaging. Also, by using the combined X-ray image together with an X-ray image other than the X-ray images used in the combining, the number of X-ray images used in spectral imaging can be increased, and a result with higher precision can be expected. Furthermore, images and spectrums that are appropriate for computation processing of spectral imaging can be obtained by performing addition/subtraction with respect to radiation images with different radiation energies, as well as addition/subtraction with respect to the spectrums thereof, in a stage before the gain correction.

<Second Embodiment

A second embodiment describes a configuration in which X-rays that change in energy during one shot are emitted, and a material characteristic image is generated using a plurality of X-ray images that are obtained by detecting the X-rays multiple times with use of a time-division method during this one shot. A radiation imaging system according to the second embodiment is composed of an X-ray generating apparatus 101, an X-ray control apparatus 102, an imaging control apparatus 103, and an X-ray imaging apparatus 104, similarly to the first embodiment (FIG. 1). Furthermore, the basic processing procedure related to the combining of X-ray images and the generation of a material characteristic image in the imaging control apparatus 103, is also similar to the first embodiment. The imaging control apparatus 103 of the second embodiment obtains a more accurate material characteristic image by increasing the accuracy of X-ray spectrums with respect to X-ray images obtained through time-division driving with two central colors.

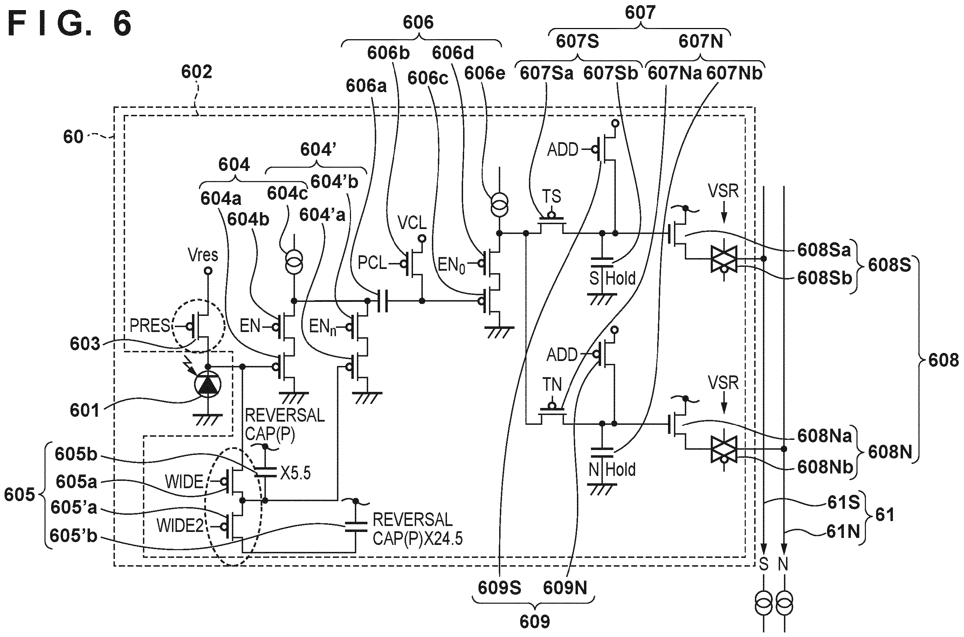

FIG. 6 shows an equivalent circuit diagram of a pixel 60 included in the X-ray imaging apparatus 104 according to the second embodiment. The pixel 60 includes a photoelectric conversion element 601 and an output circuit unit 602. The photoelectric conversion element 601 can typically be a photodiode. The output circuit unit 602 includes an amplification circuit unit 604, a clamp circuit unit 606, a sample and hold circuit unit 607, and a selection circuit unit 608.

The photoelectric conversion element 601 includes a charge accumulation unit. This charge accumulation unit is connected to a gate of a MOS transistor 604a of the amplification circuit unit 604. A source of the MOS transistor 604a is connected to a current supply 604c via a MOS transistor 604b. The MOS transistor 604a and the current supply 604c compose a source follower circuit. The MOS transistor 604b is an enable switch that is turned ON and places the source follower circuit in an operating state when an enable signal EN supplied to a gate thereof is placed in an active level.

In the example shown in FIG. 6, the charge accumulation unit of the photoelectric conversion element 601 and the gate of the MOS transistor 604a compose the same node. This node functions as a charge-voltage conversion unit that converts the charges accumulated in this charge accumulation unit into a voltage. That is to say, a voltage V (= Q/C) defined by the charges Q accumulated in this charge accumulation unit and by the capacitor value C of the charge-voltage conversion unit is present in the charge-voltage conversion unit. The charge-voltage conversion unit is connected to a reset potential $V_{res}$ via a reset switch 603. When a reset signal PRES is placed in an active level, the reset switch 603 is turned ON, and the potential of the charge-voltage conversion unit is reset to the reset potential $V_{res}$.

Using a clamp capacitor 606a, the clamp circuit unit 606 clamps noise that is output from the amplification circuit unit 604 in accordance with the potential of the reset charge-voltage conversion unit. That is to say, the clamp circuit unit 606 is a circuit for cancelling this noise from a signal that has been output from the source follower circuit in accordance with the charges generated in the photoelectric conversion element 601 due to photoelectric conversion. This noise includes kTC noise at the time of the reset. The clamping is carried out by placing a clamp signal PCL in a non-active level and placing a MOS transistor 606b in an OFF state, after placing the clamp signal PCL in an active level and placing the MOS transistor 606b in an ON state. The output side of the clamp capacitor 606a is connected to a gate of a MOS transistor 606c. A source of the MOS transistor 606c is connected to a current supply 606e via a MOS transistor 606d. The MOS transistor 606c and the current supply 606e compose a source follower circuit. The MOS transistor 606d is an enable switch that is turned ON and places the source follower circuit in an operating state when an enable signal ENo supplied to a gate thereof is placed in an active level.

A signal that is output from the clamp circuit unit 606 in accordance with the charges generated in the photoelectric conversion element 601 due to photoelectric conversion, is written as an optical signal into a capacitor 607Sb via a switch 607Sa when an optical signal sampling signal TS is placed in an active level. A signal that is output from the clamp circuit unit 606 when the MOS transistor 606b is placed in an ON state immediately after resetting the potential of the charge-voltage conversion unit, is a clamp voltage. This noise signal is written into a capacitor 607Nb via a switch 607Na when a noise sampling signal TN has been placed in an active level. This noise signal includes offset components of the clamp circuit unit 606. The switch 607Sa and the capacitor 607Sb compose a signal sample and hold circuit 607S, and the switch 607Na and the capacitor 607Nb compose a noise sample and hold circuit 607N. The sample and hold circuit unit 607 includes the signal sample and hold circuit 607S and the noise sample and hold circuit 607N.

Once a driving circuit unit has driven a row selection signal to place the row selection signal in an active level, the signal (optical signal) held by the capacitor 607Sb is output to a signal line 61S via a MOS transistor 608Sa and a row selection switch 608Sb. Furthermore, at the same time, the signal (noise) held by the capacitor 607Nb is output to a signal line 61N via a MOS transistor 608Na and a row selection switch 608Nb. The MOS transistor 608Sa, together with a non-illustrated constant current source provided for the signal line 61S, composes a source follower circuit. Similarly, the MOS transistor 608Na, together with a non-illustrated constant current source provided for the signal line 61N, composes a source follower circuit. The MOS transistor 608Sa and the row selection switch 608Sb compose a signal selection circuit unit 608S, and the MOS transistor 608Na and the row selection switch 608Nb compose a noise selection circuit unit 608N. The selection circuit unit 608 includes the signal selection circuit unit 608S and the noise selection circuit unit 608N.

The pixel 60 may include an addition switch 609S that adds up the optical signals of a plurality of adjacent pixels 60. During an addition mode, an addition mode signal ADD is placed in an active level, and the addition switch 609S is placed in an ON state. As a result, the capacitors 607Sb of the adjacent pixels 60 are connected to one another via the addition switch 609S, and the optical signals are averaged. Similarly, the pixel 60 may include an addition switch 609N that adds up the noises of a plurality of adjacent pixels 60. When the addition switch 609N is placed in an ON state, the capacitors 607Nb of the adjacent pixels 60 are connected to one another via the addition switch 609N, and the noises are averaged. An addition unit 609 includes the addition switch 609S and the addition switch 609N.

The pixel 60 may include a sensitivity changing unit 605 for changing the sensitivity. The pixel 60 can include, for example, a first sensitivity changing switch 605a and a second sensitivity changing switch 605'a, as well as circuit elements that accompany these switches. When a first changing signal WIDE is placed in an active level, the first sensitivity changing switch 605a is turned ON, and the capacitor value of a first additional capacitor 605b is added to the capacitor value of the charge-voltage conversion unit. As a result, the sensitivity of the pixel 60 decreases. When a second changing signal WIDE2 is placed in an active level, the second sensitivity changing switch 605'a is turned ON, and the capacitor value of a second additional capacitor 605'b is added to the capacitor value of the charge-voltage conversion unit. As a result, the sensitivity of the pixel 601 further decreases. By thus adding a function of reducing the sensitivity of the pixel 60, a larger amount of light can be received, and the dynamic range can be expanded. When the first changing signal WIDE is placed in an active level, it is permissible to cause a MOS transistor 604'a, instead of the MOS transistor 604a, to perform source follower operations by placing an enable signal ENw in an active level.

Next, the imaging operations of an X-ray imaging system according to the second embodiment will be described.

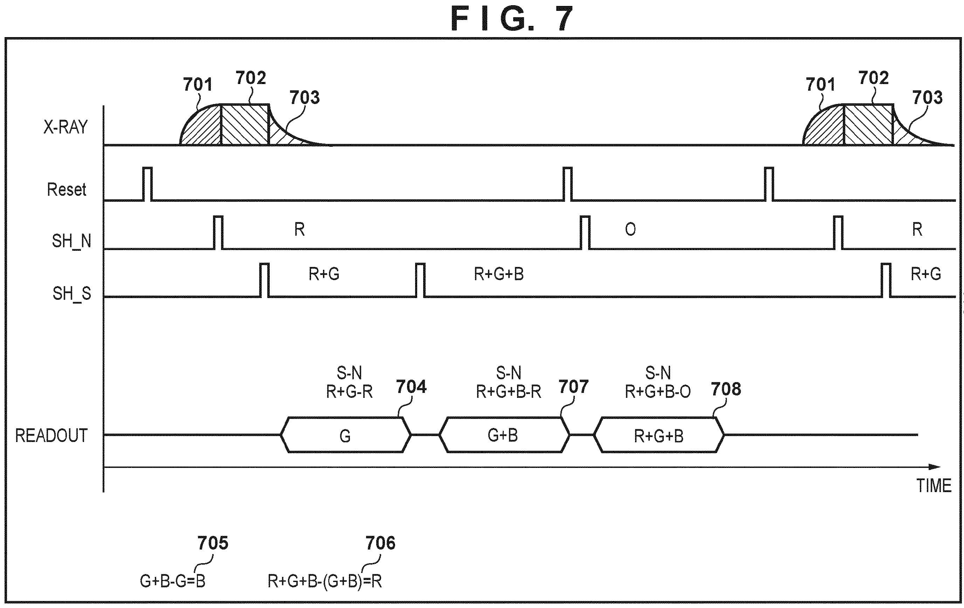

FIG. 7 shows basic driving timings of the X-ray imaging apparatus 104 for obtaining a plurality of X-ray images with different energies in the X-ray imaging system according to the second embodiment. In the second embodiment, X-rays that change in energy during one shot are emitted, and a plurality of radiation images with different X-ray energies are obtained by detecting the X-rays multiple times during that one shot. Note that in the figure, the horizontal axis represents time, and the waveforms indicate the timings of exposure to X-rays, resetting of the photoelectric conversion element 601, the sample and hold circuit 607, and readout of images from the signal line 61.

First, the photoelectric conversion element 601 is reset, which is followed by exposure to X-rays. Although the tube voltage of X-rays ideally has square waves, a limited period is required for the rise and fall of the tube voltage. Especially, when the exposure period is short with pulsed X-rays, the tube voltage is no longer deemed to have square waves, and has a waveform shown in FIG. 7. That is to say, the X-ray energy differs among three periods: a rising period (X rays 701), a stable period (X-rays 702), and a falling period (X-rays 703) of X-rays.

For this reason, sampling is performed with use of the noise sample and hold circuit 607N after exposure to the X-rays 701 in the rising period, and furthermore, sampling is performed with use of the signal sample and hold circuit 607S after exposure to the X-rays 702 in the stable period. Thereafter, the difference between the signal line 61N and the signal line 61S is read out as an image. At this time, the noise sample and hold circuit 607N holds a signal R of the X-rays 701 in the rising period, and the signal sample and hold circuit 607S holds the sum of the signal R of the X-rays 701 in the rising period and a signal G of the X-rays 702 in the stable period (R + G). Therefore, based on the aforementioned difference, an image 704 corresponding to the signal G of the X-rays 702 in the stable period is read out.

After the exposure to the X-rays 703 in the falling period and the readout of the image 704 have been completed, sampling is performed again with use of the signal sample and hold circuit 607S, and the difference between the signal line 61N and the signal line 61S is read out as an image. At this time, the noise sample and hold circuit 607N holds the signal R of the X-rays 701 in the rising period. Also, at this timing, the signal sample and hold circuit 607S holds the sum of the signal R of the X-rays 701 in the rising period, the signal G of the X-rays 702 in the stable period, and the signal B of the X-rays 703 in the falling period (R + G + B). Therefore, an image 707 corresponding to an image 707 corresponding to the sum of the signal G of the X-rays 702 in the stable period and the signal B of the X-rays 703 in the falling period (G + B) is read out.

After the readout of the image 707 has been completed, the photoelectric conversion element 601 is reset, sampling is performed again with use of the noise sample and hold circuit 607N, and the difference between the signal line 61N and the signal line 61S is read out as an image. At this time, the noise sample and hold circuit 607N holds a signal of a state where there is no exposure to X-rays, and the signal sample and hold circuit 607S holds the sum of the signal R of the X-rays 701 in the rising period, the signal G of the X-rays 702 in the stable period, and the signal B of the X-rays 703 in the falling period. Therefore, an image 708 corresponding to the sum of the signal R of the X-rays 701 in the rising period, the signal G of the X-rays 702 in the stable period, and the signal B of the X-rays 703 in the falling period (R + G + B) is read out.

Thereafter, an image 705 corresponding to the X-rays 703 in the falling period is obtained by calculating the difference between the image 707 and the image 704. Furthermore, an image 706 corresponding to the X-rays 701 in the rising period is obtained by calculating the difference between the image 708 and the image 707. In the foregoing manner, the image 706, the image 704, and the image 705 that respectively correspond to the rising period, the stable period, and the falling period of pulsed X-rays are obtained. Here, the energies of X-rays that have been used in the exposure during image formation of the images 704, 705, and 706 differ from one another. For example, computation of spectral imaging can be performed by using the image obtained by adding the image 705 and the image 706 (R + B) as a low-energy image, and the image 704 as a high-energy image (G). In this case, the spectrum obtained from the difference image based on the image 708 and the image 704 may be used as the spectrum of the low-energy image (R + B). On the other hand, in the second embodiment, the precision of the generated material characteristic image is improved by using a combined spectrum obtained by combining respective spectrums of the image 705 and the image 706.

A material separation image or a material identification image is obtained by solving the simultaneous equations with use of [Math. 1] or [Math. 2] with respect to the images 704, 705, and 706. As stated, in the second embodiment, the simultaneous equations are set by using the image obtained by combining the image 705 and the image 706 as a low-energy image, and the image 704 as a high-energy image. That is to say, the image 705 is used as an X-ray image $X_1$ based on a first X-ray energy, the image 706 is used as an X-ray image $X_2$ based on a second X-ray energy, and these are combined to generate an X-ray image $X_3$ based on a third X-ray energy.

The spectrums of X-rays of respective images are necessary in setting the simultaneous equations. As described in the first embodiment, in order to obtain the spectrum of X-rays, information of the energy of X-rays at the time of capturing of an image is necessary. In the second embodiment, as the energy changes temporally, it is difficult to obtain from the setting value of the tube voltage of the X-ray control apparatus 102. For this reason, the energy of X-rays (equivalent to a tube voltage of oo kV) that have been used in the exposure in relation to the images 704 to 706 is estimated from the X-ray transmittance of a reference object with a known linear attenuation coefficient.

The X-ray image $X_3$ corresponding to the third X-ray energy, which is a combined image, can be obtained simply by adding the X-ray image $X_1$ (image 705) obtained with the first X-ray energy and the X-ray image $X_2$ (image 706) obtained with the second X-ray energy. On the other hand, as described in the first embodiment, the X-ray spectrum of the third X-ray energy is obtained by converting the X-ray spectrums of the first and second X-ray energies into the numbers of photons, adding the numbers of photons, and standardizing the total number of photons. The X-ray spectrum obtained through the combining is more accurate than the spectrum obtained by estimating the X-ray energy with respect to the post-combination X-ray image $X_3$ corresponding to the third X-ray energy. As a result, the precision of [Math. 1] or [Math. 2] increases, and it is expected that the precision of material separation be improved, and the noise of an effective atomic number image and an area density image be reduced.

Note that in generating the combined image $X_3$ and the combined spectrum by performing addition/subtraction with respect to $X_1$ and $X_2$, multiplication by an arbitrary coefficient $\alpha$ may be performed similarly to the first embodiment. It can be said that the second embodiment is the case where the coefficient $\alpha$ in $X_3 = X_2 - \alpha X_1$ of the first embodiment takes a negative value.

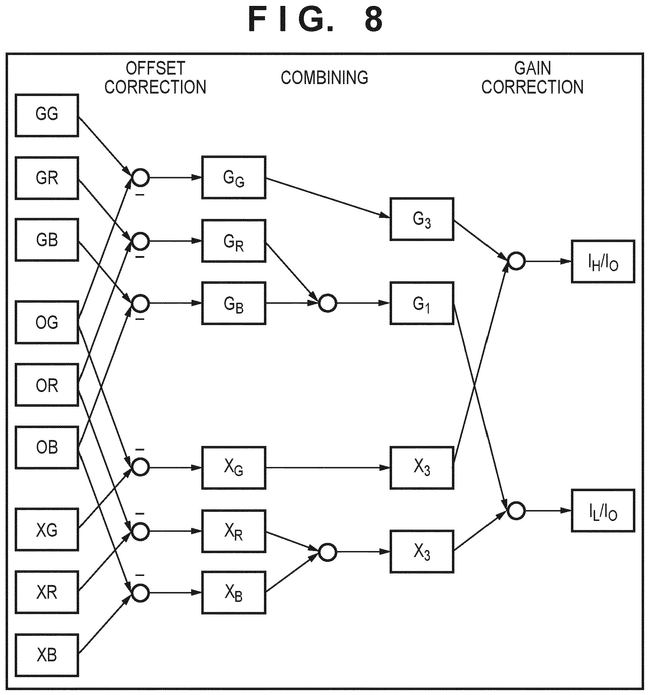

FIG. 8 is a diagram for describing the aforementioned processing performed by the imaging control apparatus 103 of the second embodiment. Images XG, XB, and XR respectively correspond to the images 704, 705, and 706 that have been obtained by emitting X-rays under the presence of a subject. Furthermore, gain correction images GG, GB, and GR are obtained by obtaining the images 704, 705, and 706 by emitting X-rays with no presence of a subject. Similarly, offset correction images OG, OB, and OR are obtained by performing driving for obtaining the images 704, 705, and 706 in a state where no X-rays are emitted. The gain correction images and the X-ray images are each corrected with use of the offset correction images; as a result, GG, $G_B$, $G_R$, XG, $X_B$, and $X_R$ are obtained. Note that although the same offset images (OG, OR, and OB) are used for the gain images (GG, GR, and GB) and the X-ray images (XG, XR, and XB) in FIG. 8, no limitation is intended by this. As described with use of FIG. 5B, it is permissible to obtain and use offset images ($O_{GG}$, $O_{GR}$, $O_{GB}$, $O_{XG}$, $O_{XR}$, and $O_{XB}$) that respectively correspond to the gain images and the X-ray images.

A third X-ray image $X_3$ is generated by combining the X-ray images $X_B$ and $X_R$ to which offset correction has been applied. Similarly, a third gain correction image $G_3$ is generated by combining the gain correction images $G_B$ and $G_R$ to which offset correction has been applied. The X-ray image XG and the gain correction image GG that have been offset are respectively used as a first X-ray image $X_1$ and a first gain correction image $G_1$. A high-energy attenuation rate image is obtained by dividing the first X-ray image $X_1$ by the first gain correction image $G_1$. Similarly, a low-energy attenuation rate image is obtained by dividing the combined third X-ray image $X_3$ by the combined third gain correction image $G_3$. The obtained attenuation rate images are used in computation of spectral imaging (generation of a material characteristic image).

As descried above, in the second embodiment, a new low-energy image is generated by combining two low-energy X-ray images that are included among three images with different X-ray energies; however, no limitation is intended by this. For example, a new high-energy image may be generated by combining (performing subtraction with respect to) an X-ray image with the highest energy and an X-ray image with intermediate energy that are included among three images with different X-ray energies, as described in the first embodiment. In this case, simultaneous equations are set by using the generated high-energy image and an image that has the lowest energy among the three images. That is to say, the second embodiment adopts a configuration in which two images included among the three images 704, 705, and 706 with different X-ray energies are combined, and two simultaneous equations are set based on the image obtained through the combining and the image that has not been used in the combining.

Also, although the second embodiment has been described in relation to a mode in which two simultaneous equations are set based on X-ray images with three different energies, the present invention is not limited to this mode. It is permissible to increase sampling nodes, and combine the spectrums of images with three or more different energies as well as the images. Furthermore, it is permissible to set simultaneous equations with use of images with three or more different energies. Moreover, it is permissible to combine the spectrums of images with two different energies as well as the images, and set two simultaneous equations with use of the combined image and one of the original images. In addition, it is permissible to apply processing described in the first embodiment while using the image 704 (G) as a high-energy image, and an image obtained by subtracting the image 704 from the image 708 (R + B) as a low-energy image.

As described above, according to each of the aforementioned embodiments, images and spectrums that are appropriate for computation processing of spectral imaging are obtained by performing addition/subtraction with respect to radiation images with different radiation energies, as well as addition/subtraction with respect to the spectrums thereof, in advance.

<Other Embodiments>

The present invention can be implemented by processing of supplying a program for implementing one or more functions of the above-described embodiments to a system or apparatus via a network or storage medium, and causing one or more processors in the computer of the system or apparatus to read out and execute the program. The present invention can also be implemented by a circuit (for example, an ASIC) for implementing one or more functions.

The present invention is not limited to the above embodiments and various changes and modifications can be made within the spirit and scope of the present invention. Therefore, to apprise the public of the scope of the present invention, the following claims are made.

The present application claims priority from Japanese Patent Application No. 2019-109018 filed June 11, 2019, which is hereby incorporated in its entirety by reference herein.

**Claims**

1. An image processing apparatus that generates a material characteristic image with use of a plurality of radiation images that have been captured with different radiation energies, **characterized by** comprising:

   combining means for combining radiation images based on two or more radiation images that are included among the plurality of radiation images, and combining radiation spectrums based on two or more radiation spectrums of the two or more radiation images; and
   generating means for generating a material characteristic image with use of two pairs of a radiation image and a radiation spectrum obtained from the plurality of radiation images,
   wherein at least one of the two pairs is a pair of the radiation image and the radiation spectrum combined by the combining means.

2. The image processing apparatus according to claim 1, **characterized in that**

   the combining means combines radiation images and radiation spectrums based on a first radiation image captured with a first radiation energy and a second radiation image captured with a second radiation energy that is higher than the first radiation energy, and
   the generating means generates a material characteristic image with use of the first radiation image or the second radiation image, the combined radiation image, and radiation spectrums thereof.

3. The image processing apparatus according to claim 2, **characterized in that**

   the combining means generates a radiation image by subtracting the first radiation image from the second radiation image, and generates a radiation spectrum by subtracting the radiation spectrum of the first radiation image from the radiation spectrum of the second radiation image, and
   the generating means generates a material characteristic image with use of the second radiation image, the combined radiation image, and radiation spectrums thereof.

4. The image processing apparatus according to claim 1, **characterized in that**

   the combining means combines radiation images and radiation spectrums based on a first radiation image captured with a first radiation energy and a second radiation image captured with a second radiation energy that is higher than the first radiation energy, and
   the generating means generates a material characteristic image with use of a third radiation image, the combined radiation image, and radiation spectrums thereof, the third radiation image having been captured with a third radiation energy different from the first radiation energy and the second radiation energy.

5. The image processing apparatus according to claim 4, **characterized in that** the third radiation energy is lower than the first radiation energy.

6. The image processing apparatus according to claim 1, **characterized in that**

   the plurality of radiation images include first and second radiation images that have been captured with first and second radiation energies, as well as a third radiation image that has been captured with a third radiation energy higher than the first and second radiation energies,
   the combining means combines radiation images and radiation spectrums based on the first and second radiation images, and
   the generating means generates a material characteristic image with use of the third radiation image, the combined radiation image, and radiation spectrums thereof.

**7.** The image processing apparatus according to any one of claims 1 to 6, **characterized in that**
the combining means combines radiation spectrums of the two or more radiation images so that the combined radiation image has a correlation with the combined radiation spectrum.

**8.** The image processing apparatus according to claim 7, **characterized in that**
the combining means performs addition/subtraction with respect to the radiation spectrums of the two or more radiation images based on respective radiation doses for the two or more radiation images at the time of imaging.

**9.** The image processing apparatus according to claim 7 or 8, **characterized in that**
the combining means combines the two or more radiation spectrums by converting the radiation spectrums into the numbers of photons based on doses for the two or more radiation images at the time of imaging, and by performing addition/subtraction.

**10.** The image processing apparatus according to any one of claims 1 to 9, **characterized in that**
the combining means multiplies at least one of the two or more radiation images and the radiation spectrums thereof by a predetermined coefficient.

**11.** The image processing apparatus according to claim 10, **characterized in that**
the coefficient is set so that the combined radiation spectrum does not have a negative value.

**12.** The image processing apparatus according to any one of claims 1 to 11, **characterized in that**
the combining means combines the two or more radiation images before gain correction for correcting gain variations on a per-pixel basis.

**13.** The image processing apparatus according to any one of claims 1 to 12, **characterized in that**
the plurality of radiation images are obtained by emitting radiation that changes in energy during one shot, and by detecting the radiation multiple times during the one shot.

**14.** The image processing apparatus according to claim 13, **characterized in that**

the plurality of radiation images are three radiation images that are obtained from three periods included in the one shot, the three periods including a rising period, a stable period, and a falling period of radiation used in exposure,
the combining means combines radiation images and radiation spectrums based on two radiation images obtained from the rising period and the falling period, and
the generating means generates a material characteristic image with use of the radiation image obtained from the stable period, the combined radiation image, and radiation spectrums thereof.

**15.** An image processing method that generates a material characteristic image with use of a plurality of radiation images that have been captured with different radiation energies, **characterized by** comprising:

a combining step of combining radiation images based on two or more radiation images that are included among the plurality of radiation images, and combining radiation spectrums based on two or more radiation spectrums of the two or more radiation images; and
a generating step of generating a material characteristic image with use of two pairs of a radiation image and a radiation spectrum obtained from the plurality of radiation images,
wherein at least one of the two pairs is a pair of the radiation image and the radiation spectrum combined in the combining step.

**16.** A program for causing a computer to execute each step of the image processing method according to claim 15.

# F I G. 1

**101**

X-RAY
GENERATING
APPARATUS

**102**

X-RAY CONTROL
APPARATUS

X-RAY IMAGING
APPARATUS

PHOSPHORS

**103**

IMAGING CONTROL
APPARATUS

**104**          **105**

OBTAIN IMAGES

CONTROL

**60**

PIXEL

**106**

TWO-DIMENSIONAL
DETECTOR

# F I G. 2

201 — SPECTRUM OF FIRST
X-RAY ENERGY

RATIO

ENERGY

202

SPECTRUM OF SECOND
X-RAY ENERGY

# F I G. 3

NUMBER OF
PHOTONS OF
X-RAY

NUMBER OF PHOTONS
OF SECOND X-RAY
ENERGY

302

ENERGY

301 — NUMBER OF PHOTONS OF
FIRST X-RAY ENERGY

303 — NUMBER OF PHOTONS OF
THIRD X-RAY ENERGY

# F I G. 4A

401～AVERAGE ENERGY OF
FIRST X-RAY ENERGY

RATIO

～201

ENERGY

# F I G. 4B

402～AVERAGE ENERGY OF
SECOND X-RAY ENERGY

RATIO

～202

ENERGY

# F I G. 4C

403～AVERAGE ENERGY OF
THIRD X-RAY ENERGY

RATIO

SPECTRUM OF
THIRD X-RAY
404～ ENERGY

ENERGY

# FIG. 5A

```
        ┌─────────────┐
        │    START    │
        └─────────────┘
               │
               ▼
┌──────────────────────────────┐
│        OBTAIN IMAGES         │──── S501
└──────────────────────────────┘
               │
               ▼
┌──────────────────────────────┐
│       OFFSET CORRECTION      │──── S502
└──────────────────────────────┘
               │
               ▼
┌──────────────────────────────┐
│     DERIVE X-RAY SPECTRUMS   │──── S503
└──────────────────────────────┘
               │
               ▼
┌──────────────────────────────┐
│    COMBINE SPECTRUMS/IMAGES  │──── S504
└──────────────────────────────┘
               │
               ▼
┌──────────────────────────────┐
│       GAIN CORRECTION        │──── S505
└──────────────────────────────┘
               │
               ▼
┌──────────────────────────────┐
│   COMPUTE SPECTRAL IMAGING   │──── S506
└──────────────────────────────┘
               │
               ▼
        ┌─────────────┐
        │     END     │
        └─────────────┘
```

# F I G. 5B

# FIG. 6

EP 3 977 936 A1

# FIG. 7

EP 3 977 936 A1

# FIG. 8

| INTERNATIONAL SEARCH REPORT | International application No. |
|---|---|
| | PCT/JP2020/021186 |

A. CLASSIFICATION OF SUBJECT MATTER
A61B 6/00(2006.01)i; G01N 23/04(2018.01)i; G01N 23/087(2006.01)i
FI: G01N23/087; A61B6/00 333; A61B6/00 350S; A61B6/00 350P; G01N23/04

According to International Patent Classification (IPC) or to both national classification and IPC

B. FIELDS SEARCHED

Minimum documentation searched (classification system followed by classification symbols)
A61B6/00; G01N23/04; G01N23/087

Documentation searched other than minimum documentation to the extent that such documents are included in the fields searched
Published examined utility model applications of Japan 1922–1996
Published unexamined utility model applications of Japan 1971–2020
Registered utility model specifications of Japan 1996–2020
Published registered utility model applications of Japan 1994–2020

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | WO 2019/044241 A1 (CANON INC.) 07.03.2019 (2019-03-07) | 1–16 |
| A | JP 2011-519031 A (CROMEK LIMITED) 30.06.2011 (2011-06-30) | 1–16 |
| A | JP 2010-537163 A (DURHAM SCIENTIFIC CRYSTALS LIMITED) 02.12.2010 (2010-12-02) | 1–16 |
| A | JP 2018-534032 A (KONINKLIJKE PHILIPS N.V.) 22.11.2018 (2018-11-22) | 1–16 |
| A | JP 2010-243369 A (TOSHIBA CORP.) 28.10.2010 (2010-10-28) | 1–16 |

☐ Further documents are listed in the continuation of Box C.     ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 14 August 2020 (14.08.2020) | 01 September 2020 (01.09.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

INTERNATIONAL SEARCH REPORT
Information on patent family members

International application No.

PCT/JP2020/021186

| Patent Documents referred in the Report | Publication Date | Patent Family | Publication Date |
|---|---|---|---|
| WO 2019/044241 A1 | 07 Mar. 2019 | JP 2019-42161 A | |
| JP 2011-519031 A | 30 Jun. 2011 | US 2011/0116605 A1<br>WO 2009/130491 A1 | |
| JP 2010-537163 A | 02 Dec. 2010 | US 2010/0220835 A1<br>WO 2009/024817 A1<br>WO 2008/142446 A2 | |
| JP 2018-534032 A | 22 Nov 2018 | US 2018/0256117 A1<br>WO 2017/060133 A1<br>CN 108135558 A | |
| JP 2010-243369 A | 28 Oct. 2010 | (Family: none) | |

Form PCT/ISA/210 (patent family annex) (January 2015)

**EP 3 977 936 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2017122705 A
- JP 2019109018 A